# EUROPEAN PATENT APPLICATION

(11) **EP 1 400 591 A1**
(43) Date of publication of application: **24.03.2004**
(21) Application number: 03078061.3
(22) Date of filing: 09.01.1998
(51) Int. Cl.: C12N 15/24, C12N 5/10, A01K 67/027, A61K 39/395, C07K 16/28, G01N 33/15, A61K 48/00, A61K 31/20, A61K 31/57

(54) **Agent for treatment of exocrine gland disorders and transgenic mouse useful for screening the same**

(30) Priority: 10.01.1997 JP 27321997
(62) Divisional of application: 98900189.6
(71) Applicant: R-Tech Ueno, Ltd., Osaka-shi, Osaka 541-0043 (JP)
(72) Inventor: Kazuo, Tsubota, Funabashi-shi Chiba 273-0031 (JP); Ichiro, Saito, Naruto-shi Tokushima 772-0000 (JP)
(74) Representative: Atkinson, Peter Birch

(57) **Abstract**

Described is a plasmid comprising the following restriction map: 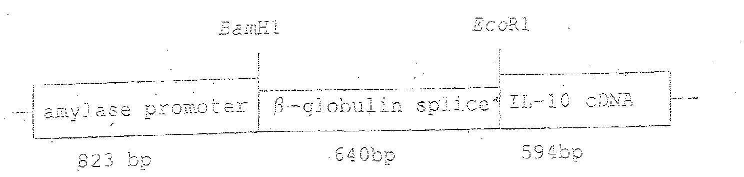 The plasmid is obtainable by ligating 640bp of splicing region of rabbit beta globulin to 823bp of human amylase promoter and 594bp of Interleukin-10 cDNA to the 3' terminal of the splicing region.

## Description

### Technical Field and Background Art

The present invention relates to an agent useful for treatment of exocrine gland disorders such as dry eye and dry mouse syndromes, and a transgenic mouse useful for said screening.

At the present state, treatments for exocrine gland disorders, such as dry eye and dry mouth syndromes, are limited to symptomatic treatments for example administration of artificial tears, and therefore, there is need for development of a radical treatment method. Various diseases accompanied by exocrine gland disorders of various pathogenesis have been known including Sjögren' s syndrome. Sjögren's syndrome is an organ specific autoimmune disease, and is characterized by inflammatory cell infiltration around the acinus or ducts of lacrimal and salivary glands, followed by destruction and atrophy of acinus or ducts epithelial cells that causes to xerosis. However, the etiology of Sjögren's syndrome remains to be determined and any substance, which is immediately responsible for the destruction of the acinus or ducts epithelial cells, has not yet been known.

Based on in vitro studies, it has been suggested that interleukin-10 (IL-10) may play a role in some autoimmune diseases. However, the in vivo role of IL-10 still remains to be determined. Further, a possibility that the production of IL-10 resulting from Epstein Barr virus infection may be implicated in the etiology of Sjögren's syndrome has been also reported.

### SUMMARY OF THE INVENTION

The present invention relates to an agent for treatment of exocrine gland disorders, a screening method for said agent and a transgenic mouse useful for said screening.

Thus, the present invention provides an agent for treatment of exocrine disorders comprising a substance which inhibits the production and/or an activity of interleukin-10 (herein after, referred as "IL-10") as an active ingredient.

Further, the present invention provides a transgenic mouse which expresses IL-10 specifically in its exocrine glands, and useful for screening agents that are potentially useful for treatment of exocrine gland disorders caused by IL-10.

Thus, the present invention provides a plasmid consisting of human amylase promoter (823 bp) splicing region of rabbit beta globulin (649 bp) ligated thereto, and mouse IL-10 cDNA ligated to the 3'-terminal of the splicing region having the restriction map as follows:

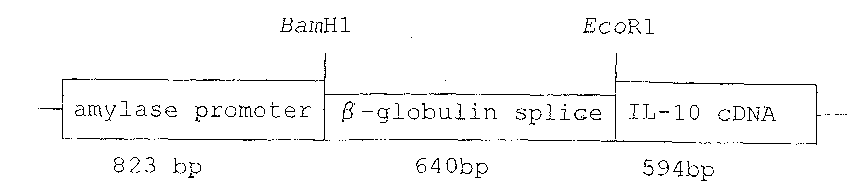

; and transgenic mice carrying said plasmid.

Resent studies suggest that IL-10 may play a significant role in the etiology of some autoimmune diseases, the inventors had studied on the role of IL-10 in the onset of Sjögren's syndrome and found that IL-10 production is directly responsible for the exocrine gland disorders.

The term "exocrine gland" used herein includes lacrimal gland, salivary gland, secretion gland in nasal mucosa or air way mucosa, gastric gland, intestinal gland, pancreatic gland, vaginal gland, sudoriferous gland and the like.

The term "treatment" or "treating" used herein refers to any means of control of a condition, including preventing the condition, curing the condition, relieving the condition and arresting or relieving the development of the condition.

The term "substance which inhibits the production and/or an activity of IL-10" used herein refers a substance which inhibits production of IL-10 in vivo, or a substance which inhibits any of various activities of IL-10. Examples of said substance include anti-Fas ligand antibody.

A variety of "activities of IL-10" are known in the art including, for example, inhibiting various cytokine production by T-cells or NK-cells; inhibiting proliferation of T-cells; inhibiting activation of monocyte/macrophage by a cytokine such as interleukin-1β or tumor necrosis factor α; inducing B-cell proliferation and differentiation through enhancement of MHC class II antigen expression; inducing cell apoptosis; inducing expression of intercellular adhesion molecules on endothelial cells. In addition, as described below in detail, the inventors found that IL-10 induces cell apoptosis through enhancement of the expression of Fas-ligands on lymphocytes, and this apoptosis causes destruction of exocrine gland. Therefore, the term "an activity of IL-10" used in this specification and claims includes enhancement of the Fas-ligand expression on lymphocytes, as well as inducement of cell apoptosis by the Fas/Fas-ligand.

The present inventor generated transgenic mice expressing IL-10 specifically in their salivary and lacrimal glands. By means of the transgenic mice, the inventor found that IL-10 plays an immediate role in the etiology of exocrine gland disorders.

### 1) The production of IL-10 transgenic mouse

Human salivary amylase promoter has been known to be expressed specifically in salivary gland but not in pancreas. Therefore, in order to express IL-10 exclusively in exocrine glands, we constructed a plasmid comprising the amylase promoter and mouse IL-10 cDNA. The plasmid was prepared by ligating the splicing region of rabbit beta globulin (640 bp) to the human salivary amylase promoter (823 bp) , and ligating the mouse IL-10 cDNA to the 3' end of the splicing region. The human salivary amylase promoter (823bp) is available from Mr. C-N, Ting, the author of *Genes and Development* 6 p1457 (1992).

The rabbit beta globulin splicing region (640bp) can be prepared from gene sequence disclosed in Hardison, R.C., Butler, and E.T. III. Lacy, E., Maniatis, T, Rosental N, *Cell* 18(4) 1285-1297(1979) and van Ooyen, A, van den Berg, J., Nabreu B., and Weissmann, C., Science 206 (4416) , 337-344 (1979) by means of PCR amplification of the sequence.

The mouse IL-10 cDNA has been deposited to ATCC under the accession number of ATCC 68027 and is available.

Thus obtained plasmid was purified and about 500 copies were introduced into a fertilized oocyte of C57BL/6 mice. Then, the oocyte was transplanted into the uterus of a pseudopregnant mouse and allowed to birth. Thus obtained mice were F₀ of the transgenic mice and they were further bred for analysis. The plasmid may be purified by introducing the same into a selectable marker gene such as pBR322.

In this manner, IL-10 may also be expressed specifically in any exocrine glands other than salivary gland as well as salivary gland.

### i) Organ specific expression of IL-10

RNAs were isolated from each of all organs, including exocrine glands of thus obtained transgenic mice according to a conventional method and analyzed by northern blotting method to determine if IL-10 was expressed in the respective organs. At the RNA level, it was confirmed that IL-10 was expressed specifically in lacrimal and salivary glands.

### ii) IL-10 expression at the protein level

In order to determine the expression of IL-10 at the protein level, tissues of lacrimal and salivary glands isolated from either transgenic or normal mice were subjected to tissue culture. The amount of IL-10 in the respective culture supernatant was determined by means of ELISA method. It was confirmed at the protein level that the production of IL-10 by both of lacrimal and salivary glands of the transgenic mice was significantly increased than that of normal mice.

### iii) Histological analysis

All organs isolated from the transgenic mice were analyzed histologically. Significant infiltration of monocyte accompanying tissue destruction was found in lacrimal and salivary glands, but no significant change was observed in the other organs. An immunohistological analysis showed that the infiltrated cells were predominantly⁻CD4+ T cells and included some CD8+ and CD21+ cells. Further, tissue samples from each organs were analyzed histopathologically by means of Tunel's method to determine if apoptosis is involved in the tissue destruction process. According to this analysis, Tunnel-positive cells were found in the lacrimal and salivary glands epithelial cells of the transgenic mice showing that the destruction found in these areas were resulting from apoptosis. Whereas, there were few Tunel-positive cells found in the normal mice without tissue destruction.

### iv) Studies on Secretion disorders

In order to study in vivo exocrine gland disorders of the transgenic mice, the secretion amount of tear and saliva in response to stimulation with the muscarinic agonist which stimulates secretion from the glands. Significantly lower amount of tear and saliva than the normal mice was observed in 8 weeks old transgenic mice. Dry nose was also found in the transgenic mice.

### v) Studies on contribution of autoantibody

In order to analyze the contribution of autoantibody and T-cell receptor in the gland disorders, T-cell clonotype were determined according to a conventional method. T-cells infiltrated into the lacrimal and salivary glands of the transgenic mice were analyzed by means of reverse transcriptase-polymerase chain reaction (RT-PCR) and single strand conformation polymorphism (SSCP). No bias was detected in any specific V *β* isolated from the lacrimal or salivary gland of transgenic mice of 6 weeks old. No clonotype was determined by SSCP. Substantially the same results were obtained with the mice of 20 or 50 weeks old. Further, no autoantibody was detected in the serum of the transgenic mice.

From the above results, it is suggested that the tissue destruction found in the lacrimal and salivary glands of the transgenic mice may be caused by autoimmune-antigen independent immune response. The inventors further studied the mechanism of the tissue destruction.

### vi) Expression of Fas-ligand

It has been reported that Fas/Fas-ligand interaction may be implicated in the mechanism of antigen independent cell apoptosis. In order to study the effect of Fas/Fas-ligand on lacrimal and salivary glands of the transgenic mice, monocytes were isolated from salivary gland of the transgenic mouse and analyzed with flow-cytometry. The obtained result was compared with that of spleen cells of the same transgenic mouse, and found that significantly higher number of Fas ligand/CD4 cells were contained in the salivary gland monocytes than the spleen cells.

### vii) Effect of IL-10 on the expression of Fas ligand

In order to study the role of IL-10 in Fas-ligand expression, normal mouse spleen cells were isolated, treated with mouse recombinant IL-10 in vitro and then analyzed with flow-cytometry 48 hours after the treatment. Significantly higher amount of Fas ligand expression was found in IL-10 stimulated cells than non-stimulated cells.

### 2) Mechanism of etiology of exocrine gland disorders

Recently, it has been reported that IL-10 enhances topical expression of cell adhesive molecules. Combining this report and the results shown here, the mechanism of the development of exocrine gland disorders at any site including Sjögren's syndrome can be suggested as follows: expression of IL-10 enhances lymphocytes infiltration to the region of the expression, IL-10 acts further on the infiltrated lymphocytes to enhance the expression of Fas-ligand, apoptosis is induced through Fas/Fas-ligand interaction and then, exocrine gland epithelial cells are damaged.

### 3) Agent of the present invention

Accordingly, it can be concluded that the exocrine gland disorders accompanying tissue destruction may be treated by interrupting the above pathway involving IL-10, that is, by inhibiting the production and/or an activity of IL-10, and/or by inhibiting expression and/or an activity of Fas/Fas-ligand. Therefore, the agent for treatment of exocrine gland disorders comprising a substance which inhibit the production and/or an activity of IL-10 as an active ingredient is effective for not only lacrimal and salivary gland disorders due to Sjögren's syndrome but also any conditions accompanying any of various exocrine gland disorders.

The conditions accompanying exocrine gland disorders which may be treated with the agent of the present invention include, for example, xerotic conditions such as Sjögren's syndrome, dry eye, dry mouse, dry nose, dry skin, and dry vagina, and chronic pancreatitis, gastritis and bronchitis due to decrease of exocrine secretion.

Examples of substances useful for the present invention which inhibit the production of IL-10 include IL-10 anti-sense such as IL-10 mRNA including Mouse IL-10 mRNA (*Science* 248, 1230-1234, 1990) and Human IL-10 mRNA (*proc* *Natl. Acad.* Sci. U.S.A. 88(4), 1172-1176, 1991), IL-10 genes such as Mouse IL-10 gene (*J. Immunol.* 148, 3618-3623, 1992 etc.), IL-10 inhibitory factor, fluticasone and pentoxifyllene.

Examples of substances useful for the present invention which inhibit an activity of IL-10 include anti-IL-10 antibodies such as monoclonal antibodies including Rat anti-mouse IL-10 antibody and Rat IgG1 anti-mouse IL-10 antibody (*J. Exp. Med.* 179, (1) 305-310, 1994).

Examples of substances which inhibit the expression of Fas ligand include Fas-ligand anti-sense, for example Fas ligand mRNA such as Human Fas ligand mRNA (*Int. Immunol.* 6(10), 1567-1574, 1994), Human Fas ligand mRNA (J.Exp. Med. 181(10) 71-77 1995), and Mouse muscle's Fas ligand mRNA (*Cell* 76, 969-976, 1994), Fas-ligand gene, letinoic acid, and glucocorticoids such as cortisol, cortisterone, cortisone, dexamethasone and prednisolone.

Examples of the substances which inhibit an activity of Fas/Fas-ligand include anti-Fas ligand antibody, soluble Fas and metalloproteinaze inhibitor.

The agent of the present invention may be used in a form as a general pharmaceutical preparation comprising the active substance as above and pharmaceutically acceptable carrier, excipient and the like. The formulation may be selected depends on the object of the treatment from, for examples, tablet, pill, liquid, emulsion, capsule, suppository, injectable preparation, such as liquid or emulsion, ointoment and eyedrops. The amount of the active substance which inhibits the expression and/or an activity of IL-10 contained in the agent of the present invention is not limited and may be an amount effective for the treatment of exocrine gland disorders to be treated. The dosage and administration schedule may be determined based on for example the condition, age, sex and body weight of the patient to be treated.

The administration of the present agent may be undergone together with a conventional treatment for exocrine gland disorders such as artificial tear instillation.

Further, as apparent from the above described mechanism, a substance which are potentially useful for treatment of exocrine gland disorders can be selected by detecting substances which inhibit IL-10 induced Fas-ligand expression. Alternatively, an agent useful for treatment of exocrine gland disorders may be screened by referring a cytotoxic ratio obtainable by the method comprising; adding effector cells obtainable by treating said cells with IL-10 to the aliquots of exocrine gland target cell culture, incubating the resulting cell mixture under the presence of either IL-10 alone, or a mixture of IL-10 and a test substance, and detecting the number of damaged cells respectively to calculate the cytotoxic ratio.

### Example 1

### Cytotoxicity test against salivary gland epithelial cells

Spleen isolated from a B6 mouse was chopped into small pieces, the cells were collected with a syringe having 22-gage injection needle and the collected cells were passed through a meshed screen to obtain cell suspension. CD4-positive T-cells were isolated from the cell suspension by means of dynabeads to provide effector cells.

Salivary gland of the same mouse as above was immersed in DMEM medium containing 10% fetal calf serum supplemented with collagenase (750 units/ml), EDTA (0.76mg/ml) and hyaluronidase type IV (500 units/ml) to isolate the salivary gland tissue, and the obtained cells were incubated in DMEM medium containing 10% fetal calf serum for 10 to 14 days in a CO₂ incubator. The cultured cells were then analyzed with flow-cytometry and were confirmed that 85-95 % of them were epithelial cells, and were used as target cells.

The target cells were labeled with 51Cr. Half of the effector cells were treated with mouse IL-10 (0.1 mg/ml) alone, whereas the other half were treated with mice IL-10 (0.1 mg/ml) and anti mice Fas ligand (FASL) neutralizing antibody ( 10 µ g/ml). Aliquots of 1 X 10⁴ target cells were added to 20-fold of 1 X 10⁴ of thus treated effector cells respectively, and the mixtures were cultured in RPMI 1640 medium containing 10% fetal calf serum for 4 hours at 37°C to determine the cytotoxycity. Target cells without effector cells were also incubated as same manner as above.

The amount of 51Cr in the supernatant of the respective culture was measured and the cytotoxic ratio was calculated by subtracting the 51Cr amount of the target cells cultured without effector cells.
The obtained results were as follows:

| | |
|---|---|
| IL-10 added | 29.5% |
| IL-10 + anti-mouse-FASL neutralizing antibody | 7.8% |

From the above results, anti-mouse FASL neutralizing antibody was shown to be useful for treatment of IL-10 induced exocrine gland disorders, and that this test procedure is useful for screening substances that are potentially useful for treatment of IL-10 induced exocrine gland disorders.

### Example 2

### Producing Transgenic Mice

Human amylase promoter was purchased from Mr. C-N Ting (*Genes and Development* 6, p1452 (1992).

Mouse IL-10 was purchased from Mr. K. W. Moore (*Science* 248, p1230, 1990).

The promoter was digested with *Bam*H1 to provide a 828 base DNA fragment. To the *Bam*H1 cleavage site, 640 base of rabbit beta globulin splicing region was ligated by means of T4-ligase, and then, *Eco*RI digested fragment of mouse IL-10 was ligated to the 3' end of the above by means of T4-ligase to provide a plasmid with the following restriction map:

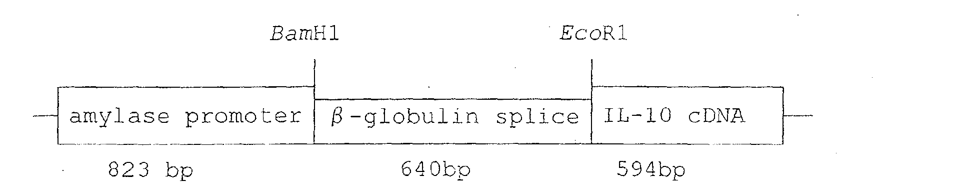

The obtained plasmid was introduced in pBR322 vector (available from Promega Corporation) to provide a gene construct. The gene construct was transferred into E-coli and amplified. Then the amplified vector was isolated and purified according to a conventional plasmid extract method. Thus obtained vector was microscopically introduced into a mouse fertilized oocyte by means of Gordon's micro injection (I.H. Gordon et. al., *Proc. Natl. Acad. Sci. USA,* 77 p7380 (1980) ) . The oocyte was then transplanted into uterus of a pseudopregnant mouse and allowed to birth. The obtained mice were designated as F₀ and the F₀ mice were further bred to provide transgenic mice.

## Claims

1. A plasmid comprising the following restriction map: 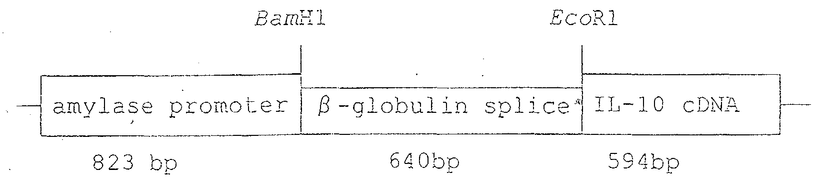 which is obtainable by ligating 640 bp of splicing region of rabbit beta globulin to 823 bp of human amylase promoter and 594 bp of IL-10 cDNA to the 3'-terminal of the splicing region.

2. A plasmid vector obtainable by inserting the plasmid of claim into vector pBR322.

3. A method for preparing a plasmid vector comprising the steps of:
ligating rabbit beta globulin splicing region to *Bam*H1 digested fragment of human exocrine gland promoter,
ligating *Eco*R1 digested fragment of mouse interleukin-10 cDNA to the 3' end of the splicing region; and
inserting the resulting plasmid into a selective marker.

4. The method according to Claim 2, wherein the respective ligating steps is performed with T4 ligase.

5. A mouse fertilized oocyte into which the plasmid vector or claim 2 is inserted.

6. A transgenic mouse which carries the plasmid gene of claim 1.

7. Use or a substance which inhibits the production and/or an activity or interlukin-10 for manufacturing pharmaceutical composition for the use of treating exocrine gland disorders.

8. The use of claim 7, wherein the substance which inhibits the production and/or an activity of interlukin-10 is anti-Fas ligand antibody.

9. A method for screening an agent for treatment of exocrine gland disorders, comprising the step of detecting cytotoxic ability of Fas-ligand expressed by interleukin-10.

10. A method for screening an agent for treatment of exocrine grand disorders, comprising the steps of:
adding effector cells, which are obtainable by treating said cells with interleukin-10, to aliquots of exocrine gland target cells;
adding to the resulting cell mixtures either interleukin-10 alone or a mixture of interleukin-10 and a substrate to be screened;
incubating the cell mixtures; and
calculating the cytotoxic ratio from the amount of damaged target cells occurs in the respective cell mixtures.
